Europäisches Patentamt

(19) European Patent Office

Office européen des brevets

(11) Publication number: **0 117 050**
**B1**

(12) **EUROPEAN PATENT SPECIFICATION**

(45) Date of publication of patent specification: **07.11.90**

(21) Application number: **84300225.4**

(22) Date of filing: **13.01.84**

(51) Int. Cl.⁵: **A 61 M 1/00, F 16 K 15/14**

(54) Intercranial pressure regulator valve.

(30) Priority: **17.02.83 US 467326**

(43) Date of publication of application:
**29.08.84 Bulletin 84/35**

(45) Publication of the grant of the patent:
**07.11.90 Bulletin 90/45**

(84) Designated Contracting States:
**DE FR GB IT NL**

(56) References cited:
**NL-C- 68 509**
**US-A-3 270 771**
**US-A-3 768 508**
**US-A-3 769 982**
**US-A-3 804 113**

(73) Proprietor: **Cordis Corporation
10555 West Flagler Street
Miami Florida 33102-5700 (US)**

(72) Inventor: **Hooven, Michael D.
6415 S.W. 116th Place (Unit G)
Miami Florida 33173 (US)**

(74) Representative: **Ford, Michael Frederick et al
MEWBURN ELLIS & CO. 2/3 Cursitor Street
London EC4A 1BQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to an intercranial pressure regulator valve and, more particularly, to a valve for and method of shunting excess cerebrospinal fluid (CSF) from a ventricle in the brain to another location in the patient's body when the pressure differential between the CSF and the other body fluid reaches a predetermined magnitude.

Hydrocephalus is a condition in which the brain is unable to relieve itself of CSF which collects in the ventricles of the brain. Such CSF, thereby, becomes excessive and results in abnormal ventricular size causing a number of adverse physiological effects including compression of the brain tissue, impairment of the blood flow in the brain tissue and of the brain's normal metabolism.

A variety of CSF pressure regulator valves and methods of controlling CSF pressure have been developed in the past which include various forms of check valves, servo valves or combinations thereof. Although these prior valves operate with some degree of success in the treatment of hydrocephalus, difficulty in the operation of such valves may be experienced due to the miniaturization of the valves and the relatively low pressures and volumes with which they must work.

US-A-3 768 508 shows a surgically implantable valve for the shunting of excess CSF when the pressure differential reaches a predetermined magnitude. The valve functions, inter alia, as an automatic non-return valve.

US-A-3 804 113 describes a non-return valve for unspecified purposes. It has a housing divided into two parts by a flexible diaphragm acted on by the fluid flowing through the valve. The diaphragm incorporates a passageway, and the area around the passageway seats against a planar surface provided on the housing, thereby to close the valve. The diaphragm is resiliently biased against this planar surface.

An intercranial pressure regulator valve for draining body fluids in accordance with the principles of the present invention minimizes the abovementioned difficulties in operation of CSF pressure regulators. In a valve of the present invention, performance is substantially improved in extremely small, miniaturized regulator valves, yet the valves are fully hydraulic in operation. In a regulator valve and method incorporating the principles of the present invention, the body fluid pressures may be accurately regulated and the valve is extremely responsive to minute changes in the pressure of the fluids. In a valve incorporating the principles of the present invention, the body fluids act on surfaces of substantial area and on both sides of a movable member or diaphragm so that the valve and method of the present invention is responsive to substantially smaller pressure differentials than the valves and methods of the prior art. In a valve of the present invention, the pressures at which response occurs may be rapidly and easily adjusted without interfering with the operation of the valve. In a valve incorporating the principles of the present invention, springs may be eliminated and a diaphragm or other movable member which is responsive to pressure variations may, instead, be employed, thereby reducing the number of parts, the possibility of malfunction and other disadvantages which may be associated with such springs.

According to the present invention there is provided a surgically implantable intercranial pressure regulator valve for regulating the flow of liquid from one location in the body to another location comprising:

a housing

a flexible diaphragm dividing the interior of said housing into first and second interior chambers with a fluid passageway through the said diaphragm from said first interior chamber to said second interior chamber;

inlet port means for establishing fluid communication between said first interior chamber and the one location;

outlet port means for establishing fluid communication between said second interior chamber and the other location;

a valve seat provided on said diaphragm for movement therewith, and defining a valving surface of said valve seat which encircles the axis of said fluid passageway;

valve closure means in said first interior chamber said valve closure means being engaged by said movable valve seat to close said passageway in response to a first lower pressure differential of the fluid acting on each side of said diaphragm, said valve seat and diaphragm then having a static position within said housing, said diaphragm and said valve seat being movable away from said valve closure means to open said passageway in response to a second higher pressure differential of the fluid acting on each side of said diaphragm;

the valve closure means providing a second valving surface encircling the axis of said fluid passageway for coacting with said valving surface of said valve seat to close said fluid passageway in response to said first lower pressure differential; and

adjusting means for adjusting the position of said valve closure means within said first chamber to vary bias with which said first valving surface engages said second valving surface and hence vary said pressure differential at which said passageway opens and flow occurs.

The valve of the invention may serve to drain cerebrospinal fluid (CSF) and may include a catheter for communicating with the tissue in the body from which the CSF is to be drained.

Preferably the aforementioned valve closure means comprises a substantially spherical ball which is engageable by the valve seat means.

An embodiment of the invention will now be described in detail. In the course of this description, reference will frequently be made to the attached drawing in which:

FIG. 1 is illustrative of a hydrocephalus system in which the preferred embodiment of intercranial pressure regulator valve of the present invention may be incorporated and the preferred method of the present invention may be practiced;

FIG. 2 is an enlarged, exploded view of a preferred embodiment of valve incorporating the principles of the present invention;

FIG. 3 is a reduced, plan view of the assembled valve shown in FIG. 2 as incorporated in the hydrocephalus treatment system shown in FIG. 1; and

FIG. 4 is an enlarged, cross-sectioned, partially broken, side elevational view of the valve as viewed substantially along line 4—4 in FIG. 3.

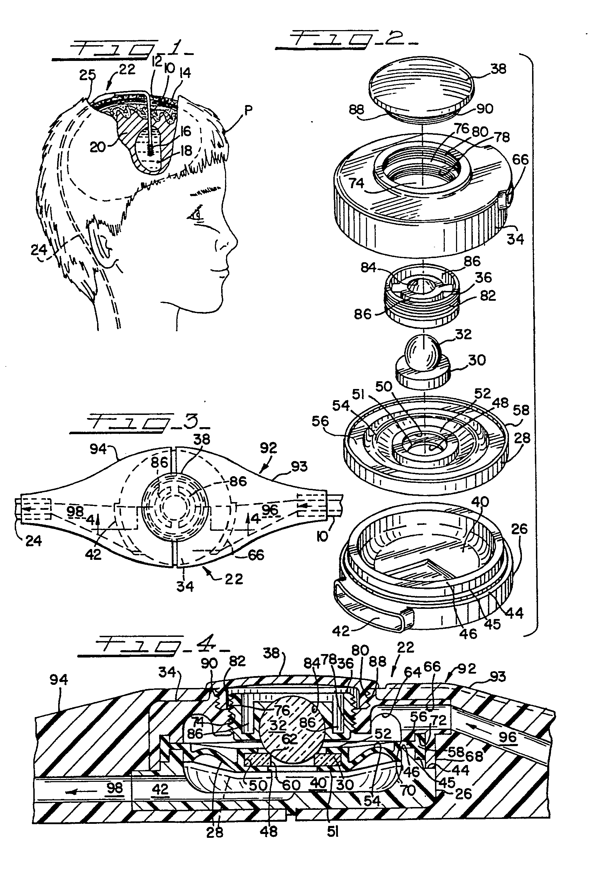

In FIG. 1 a hydrocephalus system incorporating the principles of the present invention is illustrated. The system shown includes a ventricle catheter 10 which is inserted through an opening 12 which has been formed in the skull 14 of the patient P who is to undergo the hydrocephalus treatment. The catheter 10 is preferably radio-paque. The distal end 16 of the ventricular catheter 10 is positioned in a ventricle 18 in the patient's brain tissue 20 in which the CSF accumulates. The other end of the catheter 10 is coupled to the intercranial pressure regulator valve 22 of the present invention, as shown in FIG. 1; and a drain catheter 24 is coupled to the valve 22 to receive the discharge from the valve to drain the CSF discharge to another location in the patient's body, such as the right atrium of the patient's heart (not shown). The valve 22, the portion of the ventricle catheter 10 exterior to the skull 14, and the drain catheter 24 are preferably located between the patient's skull 14 and scalp 25, as shown in FIG. 1.

The details of the valve 22 are best shown in FIGS. 2—4. As shown in FIG. 2, the preferred embodiment of pressure regulator valve incorporating the principles of the present invention includes a valve casing bottom 26, a flexible diaphragm 28, a valve seat 30 and valve closure ball 32, a valve casing top 34, a threaded screw member 36, and a casing closure cap 38. The casing bottom 26, casing top 34, screw member 36 and closure cap 38 are formed of a suitable durable, biologically compatible material, such as thermoplastic polymers of polyethersulfone or polycarbonates.

The valve casing bottom 26 comprises a substantially cup shaped member which defines, as shown in FIG. 4, a fluid discharge chamber 40 having a discharge port 42 of preferably elongate cross-section as shown in FIG. 2. The upper rim of the casing bottom 26 is formed with a plurality of stepped raised shoulders 44, 45 and 46.

The diaphragm 28 comprises a preferably substantially circular, flexible movable disc having a fluid flow passage 48 adjacent its center. An annular groove 50 is formed in the diaphragm adjacent the flow passage 48 to receive and hold the valve seat 30 as shown in FIG. 4. The groove 50 is defined by a lower flange 51 and an upper flange 52, the upper flange 52 being preferably somewhat shorter than the lower flange 51 to accommodate unrestricted movement of the diaphragm and valve seat 30 relative to the valve closure ball 32 during operation of the valve. The lower flange 51 is surrounded by an annular disc portion 54 which is preferably convoluted when installed in the casing, as shown in FIG. 4. The convolution provides increased flexibility of the diaphragm and stability against cocking during operation. A horizontal annular flange 56 surrounds the annular disc portion 54. The annular flange 56 is encircled by a generally vertical annular flange 58 to complete the diaphragm construction. The diaphragm 28 may be formed of any durable, flexible, biologically compatible material, such as Silastic rubber.

The valve seat 30 is preferably circular, as shown in FIGS. 2 and 4, and includes an opening 60 through its center to provide for the passage of fluid through the diaphragm. A suitable shoulder 62 is formed at the top of the opening 60 in valve seat 30 to engage the valve closure ball 32 to form a seal with the ball 32 to block passage of the fluid through the opening 60.

The valve closure ball 32 is positioned on the cephalad side of the diaphragm and is preferably substantially spherical in shape, as shown in FIGS. 2 and 4, although it will be understood that other shapes may be satisfactorily employed in the present invention. As shown in FIG. 4, valve closure ball 32 is solid, although it may be hollow, if desired.

Both the valve seat 30 and valve closure ball 32 are also formed of a durable, yet biologically compatible material. By way of example, sapphire may be used as a material to form the valve seat and ball.

The valve casing top 34 also comprises a substantially cup shaped member which defines, as shown in FIG. 4, an inlet chamber 64 and an inlet port 66, the latter of which is also of elongate oblong cross-section, as best seen in FIG. 2. The casing top 34 includes, as best shown in FIG. 4, a pair of downwardly extending annular flanges 68 and 70 with flange 68 being somewhat longer than flange 70. Flanges 68 and 70 are spaced from each other to define a groove 72 therebetween.

The diameter and width of flange 70 are preferably substantially equal to the diameter and width of shoulder 46 on the casing bottom 26 and overlies that shoulder when the casing is assembled, as shown in FIG. 4. The diameter and width of groove 72 are substantially equal to the diameter and width of shoulder 45 on the casing bottom and overlies that shoulder when the casing is assembled, as shown in FIG. 4. The diameter and width of flange 68 are substantially equal to the diameter and width of shoulder 44 on the casing bottom and overlies that shoulder when the casing is assembled. The vertical annular flange 58 of the diaphragm 28 is clamped between the groove 72 and shoulder 45 and the horizontal annular flange 56 of the diaphragm is clamped between the flange 70 and shoulder 46 when the valve casing is assembled, as shown in FIG. 4. The valve casings and diaphragm, thereby, fit snugly together when assembled and they are all secured together by suitable means such as solvent, adhesive or ultrasonic bonding.

An opening 74 extends through the top of the top valve casing 34. The opening is preferably stepped at 6 and the wall of the opening is threaded with two sets of threads 78 and 80.

The screw member 36 contains external threads 82 on its outside surface which are adapted to be threaded into the threads 78 in the opening 74 of the casing top. The ball 32 is attached in a recess 84 in the screw member 36 by suitable means, such as insert molding. The ball 32 is, therefore, stationarily mounted to the screw member. A pair of arcuate slots 86 are also formed in the screw member, as shown in FIG. 2, to receive a suitable tool for adjusting the extent to which the screw member is threaded into the casing top.

The closure cap 38 includes a downwardly extending annular flange 88 which contains threads 90 on its external surface so that the cap may be threaded onto the threads 80 for closure of the opening 74 in the casing top 34.

Once the lower and upper valve casings 26 and 34, the diaphragm 28, the valve seat 30, the ball 32 and the screw member 36 have been assembled together, a flexible outer housing is assembled over the valve casing by sliding the outer housing over the valve casing. This flexible outer housing 92 is preferably formed by a pair of housing half members 93 and 94, as shown in FIGS. 3 and 4. Housing half member 93 includes a tapering inlet antichamber 96 which, at its wider end, communicates with the inlet port 66 and at the other narrower end with the ventricular catheter 10. The other housing half member 94 also includes a tapered discharge antichamber 98 which, at its wider end, communicates with the discharge port 42 and at the other narrower end with the drain catheter 24. The outer housing 92 is formed of a flexible, biologically compatible material, such as Silastic rubber.

The closure cap 38 is preferably exposed through the housing 92, as shown in FIGS. 3 and 4, to allow adjustment of the screw member 36 and its ball 32 during the assembly of the valve system.

Although the operation of the intercranial pressure regulator valve and method of the present invention should be clear from the foregoing description, a brief description of a preferred operation and method of hydrocephalus treatment will be described.

The CSF in the ventricle 18 which is to be drained communicates with the valve via the ventricular catheter 10, the inlet antichamber 96 in the housing half member 93, the inlet port 66 and the inlet chamber 64. Thereby, the pressure of this CSF will act upon substantially the entire upper surface of the diaphragm 28 which is of substantial area as may be seen in FIG. 4. So long as the pressure of the fluid on the discharge side of the valve which is also acting upon the entire lower surface of the diaphragm 28 is substantially equal to the pressure of the CSF acting upon the upper surface of the diaphragm, the resilient nature of the diaphragm will cause it to flex upwardly, as viewed in FIG. 4, and cause the valve seat 30 to engage the stationary valve closure ball 32 to close the passages 48 and 60 through the diaphragm and valve seat and prevent flow of the fluid through these passages.

When a pressure increase occurs in the CSF in the ventricle, this increased pressure will be transmitted to the upper surface area of the diaphragm 28. When the pressure differential increases between the upper surface area of the diaphragm and the fluid in the discharge chamber 40 which exerts its pressure against the bottom surface area of the diaphragm, the diaphragm will begin to flex downwardly, as shown by the dot and dash lines in FIG. 4, in response to this increased differential in pressure. Such flexing will cause the valve seat 30 to move away from the stationary valve closure ball and allow CSF to pass between the upper surface of the diaphragm from chamber 64 through the passages 48 and 60 to the discharge chamber 40 and the lower surface of the diaphragm. This fluid will be discharged through the discharge port 42, discharge antichamber 98 and discharge catheter 24. The discharge of CSF through passages 48 and 60 will continue until the pressure differential between the upper and lower surfaces of the diaphragm returns to a predetermined low differential causing the diaphragm to again flex upwardly until the valve seat 30 engages the valve closure ball 32 to close the passages 48 and 60.

The pressure differential at which the valve opens may be adjusted as necessary for the patient. This adjustment may be readily accomplished by threading up or down the screw member 36 to adjust the force by which the spherical ball 32 bears against the valve seat. If the ball 32 is moved downwardly, it will preflex the diaphragm downwardly somewhat resulting in a higher pressure differential which must be reached before the valve seat 30 begins to move away from the ball 32. The converse is true if the ball is threaded upwardly, as viewed in FIG. 4.

From the foregoing description it will be seen that the valve and method of the present invention are extremely responsive to very low changes in pressure of the CSF. This responsiveness is a result of the fact that the fluid pressures which act upon the surfaces of the diaphragm are exerted over substantially the entire areas of the upper and lower surfaces of the diaphragm. Thus, very small changes in pressure differential are capable of actuating the valve. By way of example, the pressure regulator valve described herein is capable of accurate operation at CSF pressure differentials of between 0—200 millimeters of water.

Moreover, extreme sensitivity of response may be realized in pressure regulator valves of very small size, for example valves which may be only one quarter inch in thickness and three quarter inch in diameter, (approx. 6 mm thickness and 19 mm diameter).

It will also be seen that the valve incorporating the principles of the present invention may be easily and rapidly adjusted to conform with the pressure differential requirements of a given patient. Such adjustment may be accomplished without interfering in any manner with the valve operation itself.

It will also be understood that the embodiment of the present invention which has been described is merely illustrative of one of the applications of the principles of the present invention. Numerous modifications may be made by those skilled in the art without departing from the scope of the invention as defined in the claims.

**Claims**

1. A surgically implantable intercranial pressure regulator valve (22) for regulating the flow of liquid from one location in the body to another location comprising:
   a housing (26, 34)
   a flexible diaphragm (28) dividing the interior of said housing into first (64) and second (40) interior chambers with a fluid passageway through said diaphragm from said first interior chamber (64) to said second interior chamber (40);
   inlet port means (66) for establishing fluid communication between said first interior chamber (64) and the one location;
   outlet port means (42) for establishing fluid communication between said second interior chamber (40) and the other location;
   a valve seat (30) provided on said diaphragm for movement therewith, and defining a valving surface (62) of said valve seat which encircles the axis of said fluid passageway (60);
   valve closure means (32) in said first interior chamber said valve closure means being engaged by said movable valve seat (30) to close said passageway (60) in response to a first lower pressure differential of the fluid acting on each side of said diaphragm (28), said valve seat (30) and diaphragm (28) then having a static position within said housing, said diaphragm (28) and said valve seat (30) being movable away from said valve closure means (32) to open said passageway (60) in response to a second higher pressure differential of the fluid acting on each side of said diaphragm (28);
   the valve closure means (32) providing a second valving surface encircling the axis of said fluid passageway (60) for coacting with said valving surface (62) of said valve seat to close said fluid passageway in response to said first lower pressure differential; and
   adjusting means (36) for adjusting the position of said valve closure means (32) within said first chamber (64) to vary bias with which said first valving surface (62) engages said second valving surface and hence vary said pressure differential at which said passageway (60) opens and flow occurs.

2. A flow regulating valve as defined in claim 1 wherein said first valving surface (62) is biased into engagement with said second valving surface by said diaphragm (28) when said valve seat (30) is in said static position to increase the said second pressure differential at which flow occurs between said first and second interior chambers (64, 40), and said adjusting means (36) acts to vary said bias.

3. A flow regulating valve as defined in claim 1 or claim 2 wherein said adjusting means (36) move said valve closure means (32) along said axis of said fluid passageway.

4. A flow regulating valve as defined in claim 3 wherein said adjusting means (36) are operable from the exterior of said housing (26, 34).

5. A flow regulating valve as defined in claim 3 or claim 4 wherein said adjusting means include an adjustment member (36) mounted in said first interior chamber (64) for movement toward and away from said diaphragm (28) and said valve closure means (32) is mounted on said member.

6. A flow regulating valve as defined in claim 5 wherein said adjustment member comprises a screw member (36) threadably engaged to said housing (34) and rotatable from the exterior thereof.

7. A flow relating valve as defined in anyone of the preceding claims wherein said valve seat (30) and said valve closure means (32) are each formed of a hard substantially non-deformable material.

8. A flow regulating valve as defined in claim 7 wherein said hard substantially non-deformable material is sapphire.

9. The valve of any one of the preceding claims, wherein said valve closure means comprises a substantially spherical ball (32) which is engageable by said valve seat (30).

**Patentansprüche**

1. Chirurgisch implantierbares Schädeldruck-Regelventil zur Regelung der Strömung einer Flüssigkeit von einem Ort im Körper zu einem anderen Ort, umfassend:
   ein Gehäuse (26, 34);
   eine flexible Membran (28), die das Innere des besagten Gehäuses in eine erste (64) und zweite (40) Innenkammer unterteilt mit einem Flüssigkeitsdurchlaß durch diese Membran von der ersten Innenkammer (64) zu der zweiten Innenkammer (40);
   Einlaßöffnungsmittel (66), um eine Flüssigkeitsverbindung zwischen der ersten Innenkammer (64) und dem einen Ort zu schaffen;
   Auslaßöffnungsmittel (42), um eine Flüssigkeitsverbindung zwischen der zweiten Innenkammer (40) und dem anderen Ort zu schaffen;
   einen Ventilsitz (30), der an der Membran zur Bewegung mit dieser angeordnet ist und der eine Ventilfläche (62) des Ventilsitzes begrenzt, der die Achse des besagten Flüssigkeitsdurchlasses (60) umgibt;
   Ventilschließmittel (32) in der besagten ersten Innenkammer, die mit dem besagten beweglichen Ventilsitz (30) in Eingriff stehen, um den besagten Flüssigkeitsdurchlaß (60) bei einem ersten unteren Druckunterschied der auf jede Seite der Membran (28) einwirkenden Flüssigkeit zu schließen, wobei der besagte Ventilsitz (30) und die Membran (28) dann in dem Gehäuse eine statische Position einnehmen, wobei die Membran (28) und der Ventilsitz (30) bei einem zweiten höheren Druckunterschied der auf jede Seite der Membran (28) einwirkenden Flüssigkeit von den Ventilschließmitteln (32) abhebbar sind, um den Flüssigkeitsdurchlaß (60) zu öffnen;

wobei die Ventilschließmittel (32) eine die Achse des Flüssigkeitsdurchlasses (60) umgebende zweite Ventilfläche bilden zum Zusammenwirken mit der Ventilfläche (62) des Ventilsitzes (30), um den Flüssigkeitsdurchlaß bei dem ersten unteren Druckunterschied zu schließen; und

Einstellmittel (36) zum Einstellen der Lage der Ventilschließmittel (32) innerhalb der ersten Kammer (64) zum Verändern der Vorspannung, mit der die erste Ventilfläche (62) an der zweiten Ventilfläche anliegt, und damit zur Änderung des Druckunterschiedes, bei dem der Durchlaß (60) geöffnet wird und ein Überströmen erfolgt.

2. Regelventil nach Anspruch 1, wobei die erste Ventilfläche (62) zum Eingriff mit der zweiten Ventilfläche durch die Membran (28) vorgespannt ist, wenn sich der besagte Ventilsitz (30) in seiner statischen Position befindet, um den zweiten Druckunterschied zu erhöhen, bei dem eine Strömung zwischen der ersten und der zweiten Innenkammer (64, 40) erfolgt, und wobei die besagten Einstellmittel (36) zur Veränderung dieser Vorspannung dienen.

3. Regelventil nach Anspruch 1 oder 2, wobei die besagten Einstellmittel (36) die besagten Ventilschließmittel (32) längs der Achse des Flüssigkeitsdurchlasses (60) bewegen.

4. Regelventil nach Anspruch 3, wobei die Einstellmittel (36) von der Außenseite des Gehäuses (26, 34) her betätigbar sind.

5. Regelventil nach Anspruch 3 oder 4, wobei die Einstellmittel ein Einstellteil (36) umfassen, das in der ersten Kammer (64) gegenüber der Membran (28) abstandsveränderlich angeordnet ist und wobei die Ventilschließmittel (32) auf diesem Teil angeordnet sind.

6. Regelventil nach Anspruch 5, wobei das Einstellteil aus einem Gewindeteil (36) besteht, das in das Gehäuse (34) eingeschraubt und von dessen Außenseite her verdrehbar ist.

7. Regelventil nach einem der vorhergehenden Ansprüche, wobei der besagte Ventilsitz (30) und die Ventilschließmittel (32) jeweils aus einem harten, im wesentlichen nicht verformbaren Material bestehen.

8. Regelventil nach Anspruch 7, wobei das harte, im wesentlichen nicht verformbare Material ein Saphir ist.

9. Regelventil nach einem der vorhergehenden Ansprüche, wobei die Ventilschließmittel aus einer im wesentlichen sphärischen Kugel (32) bestehen, die mit dem Ventilsitz (30) in Eingriff bringbar ist.

**Revendications**

1. Valve (22) de régulation de la pression intracrânienne implantable chirurgicalement pour réguler le flux de liquide d'une localisation dans le coprs vers une autre localisation comprenant:
    un boîtier (26, 34)
    un diaphragme flexible (28) divisant l'intérieur dudit boîtier en une première (64) et une seconde (40) chambres intérieures avec un passage pour le fluide à travers ledit diaphragme de ladite première chambre intérieure (64) vers ladite seconde chambre intérieure (40);
    un moyen formant orifice d'admission (66) pour établir une communication de fluide entre ladite première chambre intérieure (64) et l'une des localisations;
    un moyen formant orifice d'émission (42) pour établir une communication de fluide entre ladite seconde chambre intérieure (40) et l'autre localisation;
    un fond de valve (30) prévu sur ledit diaphragme pour se déplacer avec, et définissant une surface de valve (62) dudit fond de valve qui encercle l'axe dudit passage (60) de fluide;
    un moyen (32) de fermeture de valve dans ladite première chambre intérieure, ledit moyen de fermeture de valve étant engagé par ledit fond de valve mobile (30) pour fermer ledit passage (60) en réponse à une première différence de pression basse du fluide agissant sur chaque côté dudit diapfragme (28), ledit fond de valve (30) et le diaphragme (28) ayant ainsi une position statique à l'intérieur dudit boîtier, ledit diaphragme (28) et ledit siège de valve (30) étant éloignables dudit moyen (32) de fermeture de valve pour ouvrir ledit passage (60) en réponse à une seconde différence de pression plus importante, du fluide agissant sur chaque côté dudit diaphragme (28);
    le moyen (32) de fermeture de valve fournissant une seconde surface de valve encerclant l'axe dudit passage (60) de fluide pour coopérer avec ladite surface de valve (62) dudit fond de valve pour fermer ledit passage de fluide en réponse à la première basse différence de pression; et
    des moyens d'ajustement (36) pour ajuster la position dudit moyen (32) de fermeture de valve à l'intérieur de ladite première chambre (64) afin de faire varier l'effet avec lequel ladite première surface de valve (62) engage ladite seconde surface de valve et de là, faire varier ladite différence de pression pour laquelle le passage (60) s'ouvre et l'écoulement se produit.

2. Une valve de régulation d'écoulement telle que définie dans la revendication 1 dans laquelle ladite première surface de valve (62) est amenée en engagement avec ladite seconde surface de valve par ledit diaphragme (28) lorsque ledit fond de valve (30) est dans la position dite statique pour augmenter la différence de pression dite seconde à laquelle se produit l'écoulement entre les chambres intérieures (64, 40) dites première et seconde, et lesdits moyens d'ajustement (36) agissent pour faire varier ledit effet.

3. Une valve de régulation d'écoulement telle que définie dans la revendi'cation 1 ou 2 dans laquelle lesdits moyen d'ajustement (36) déplacent ledit moyen (32) de fermeture de valve le long dudit axe dudit passage de fluide.

4. Une valve de régulation d'écoulement telle que définie dans la revendication 3 dans laquelle lesdits moyens d'ajustement (36) sont actionnables à partir de l'extérieur dudit boîtier (26, 34).

5. Une valve de régulation d'écoulement telle que définie dans la revendication 3 ou 4 dans laquelle lesdits moyens d'ajustement incluent un membre d'ajustement (36) monté dans ladite première chambre intérieure (64) pour se déplacer vers ledit diaphragme (28) ou s'en éloigner et ledit moyen de fermeture de valve (32) est monté sur ledit membre.

6. Une valve de régulation d'écoulement telle que définie dans la revendication 5, dans laquelle ledit membre d'ajustement comprend un membre formant vis (36) engagé de façon vissée sur ledit boîtier (34) et entraînable en rotation à partir de sa partie extérieure.

7. Une valve de régulation d'écoulement telle que définie dans l'une quelconque des revendications précédentes dans laquelle ledit fond de valve (30) et ledit moyen de fermeture de valve (32) sont chacun formés d'un matériau dur pratiquement indéformable.

8. Une valve de régulation d'écoulement telle que définie dans la revendication 7 dans laquelle ledit matériau dur pratiquement indéformable est le saphir.

9. La valve selon l'une quelconque des revendications précédentes, dans laquelle ledit moyen de fermeture de valve comprend une boule sensiblement sphérique (32) qui est engageable par ledit fond de valve (30).

FIG.1.

FIG.2.

FIG.3.

FIG.4.